# DEMANDE DE BREVET EUROPEEN

(11) **EP 2 022 509 A1**
(43) Date de publication de la demande: **11.02.2009**
(21) Numéro de dépôt: 08104934.8
(22) Date de dépôt: 31.07.2008
(51) Int. Cl.: A61L 9/12, A61L 9/14, B05B 7/06

(54) **Dispositif de diffusion d'un produit de désinfection avec nettoyage automatique et procédé de diffusion associé**

(30) Priorité: 07.08.2007 FR 0756989
(71) Demandeur: Lange, Alain, 98000 Monaco (MC)
(72) Inventeur: LANGE, Alain, 98000 Monaco (MC)
(74) Mandataire: Schmit, Christian Norbert Marie

(57) **Abrégé**

L'invention concerne un dispositif (1) de diffusion d'un produit de désinfection. Ce dispositif comporte une pompe à air (2) pour produire de l'air comprimé, un réservoir (3) de produit de désinfection, et au moins une buse d'injection (4). Selon l'invention, le dispositif comporte également un premier circuit (5) fermé et un deuxième circuit (6) fermé, le premier circuit formant un conduit relié à la pompe et destiné à être rempli d'air et le deuxième circuit formant un autre conduit relié au réservoir de produit et destiné à être rempli de produit, le premier circuit et le deuxième circuit étant raccordés l'un à l'autre par la buse d'injection, la buse recevant l'air comprimé et le produit de telle sorte que le produit contenu dans le deuxième circuit est expulsé vers l'extérieur au travers de la buse par dépression de l'air contenu dans le premier circuit et passant au travers de la buse.

## Description

### Domaine de l'invention

La présente invention concerne un dispositif de diffusion d'un produit de désinfection avec nettoyage automatique. L'invention concerne également un procédé de diffusion de ce produit. L'invention a pour but de permettre une diffusion précise du produit simultanément ou non à de multiples points, lesdits points pouvant êtres éloignés les uns des autres, et à partir d'une centrale automatique et d'un réservoir unique.

De manière générale, l'invention est adaptée aux domaines de l'amélioration de la qualité de l'air ou toute application dans laquelle il est nécessaire de diffuser un produit tout en gérant précisément la quantité diffusée en toute sécurité (décontamination des conduits d'air conditionné, traitement de l'air en élevages industriels (diffusion de produits médicamenteux), neutralisation d'odeurs).

### Etat de la technique

Il est connu un dispositif de diffusion d'un produit de désinfection de micro-organismes. Un tel dispositif est installé à chaque point d'injection. Le dispositif comporte un réservoir de produit de désinfection, au moins un injecteur de ce produit et des commandes électriques afin de déclencher la pulvérisation du produit au travers de l'injecteur. Le fait d'être obligé d'installer un dispositif complet à chaque point d'injection (commandes électriques et réservoir) entraîne un coût élevé des installations et rend les opérations de contrôle et de maintenance fastidieuses. La plupart du temps, les injecteurs se trouvent éloignés et dans des endroits peu accessibles tels que les plafonds ou gaines techniques.

### Résumé de l'invention

Dans l'invention, on prévoit un dispositif de diffusion qui permet l'installation de nombreux points d'injection distants gérés par un automate. Plus précisément, dans l'invention on a prévu un dispositif comprenant un double circuit d'alimentation disposé en boucle. Ce dispositif comprend plus particulièrement un premier circuit d'amenée d'air comprimé et un deuxième circuit d'amenée de produit, le premier circuit et le deuxième circuit étant reliés entre eux par au moins une buse d'injection. Le premier circuit et le deuxième circuit sont alimentés respectivement par une pompe à air unique et par un réservoir unique.

Le deuxième circuit réalise un cheminement en boucle du produit. En effet, le deuxième circuit prend naissance et se termine dans le réservoir.

Selon l'invention, la buse est plus précisément un endroit du dispositif où communiquent le premier circuit et le deuxième circuit l'un avec l'autre. L'air contenu dans le premier circuit subit une dépression à l'intérieur de la buse et permet ainsi, par effet Venturi, d'entraîner vers l'extérieur de la buse le produit de désinfection.

Selon l'invention, plusieurs buses sont installées et seulement un réservoir de produit de désinfection et une pompe sont nécessaires. L'invention permet ainsi de délocaliser la pompe à air et le réservoir dans un endroit autorisant un accès facilité pour leur entretien et leur contrôle, tel dans un local technique. Le réservoir et la pompe à air sont logés dans un coffret ou armoire de protection et de sécurité. Le coffret renferme également un boîtier de commande qui rassemble toute les connexions électriques nécessaires, notamment, à la commande de la pompe à air, des buses d'injection et de dispositifs de sécurité (détecteur de pression, de niveau,....).

Le dispositif selon l'invention permet d'injecter, de préférence par micro-diffusion, des quantités de produit sous forme de particules en suspension dans l'air de façon homogène, très précise instantanément par une ou plusieurs buses spécifiques pouvant être disposées jusqu'à plus de 100 mètres du coffret.

Le deuxième circuit et chaque buse du dispositif selon l'invention sont vidés et nettoyés automatiquement au moins une fois par jour à l'air comprimé. Le produit est redéversé dans le réservoir. Le nettoyage des buses permet d'éviter un dessèchement du produit de désinfection dans le conduit formé par le deuxième circuit et dans la buse et donc une obstruction du conduit et de la buse. Ce nettoyage est automatisé et permet de diminuer considérablement les opérations de maintenance.

Le dispositif permet la diffusion du produit de désinfection sans modifier sa composition.

L'invention a pour but d'être appliquée à de gros chantiers pour pouvoir, dans un bâtiment complet, injecter dans des gaines de climatisation, dans des gaines de ventilation, de manière automatique, le produit de désinfection et pouvoir aller à de multiples points sans être obligé de se déplacer.

Le produit de désinfection est injecté sous forme d'une pulvérisation dans l'air que l'on respire. L'invention permet de contrôler précisément la quantité de produit de désinfection à injecter.

Un des buts de l'invention est également de créer un circuit fermé et de pouvoir injecter à distance le produit de désinfection sous air comprimé et qui est géré à partir d'un coffret qui comprends un seul réservoir, un seul pompe à air comprimé et un seul boîtier de commande.

Il n'y a pas de perte de charge dans chacun des circuits car il n'y a pas beaucoup de débit.

Quand la diffusion est finie, le produit de désinfection n'est pas perdu, on vidange la machine.

L'invention a donc pour objet un dispositif de diffusion d'un produit de désinfection, comportant
- une pompe à air pour produire de l'air comprimé,
- un réservoir de produit de désinfection, et
- au moins une buse d'injection,
caractérisé en ce que le dispositif comporte également
- un premier circuit et un deuxième circuit, le premier circuit formant un conduit relié à la pompe et destiné à être rempli d'air et le deuxième circuit formant un autre conduit relié en boucle au réservoir de produit et destiné à être rempli de produit, le premier circuit et le deuxième circuit étant raccordés l'un à l'autre par au moins une buse d'injection, la buse recevant l'air comprimé et le produit de telle sorte que le produit contenu dans le deuxième circuit est expulsé vers l'extérieur au travers de la buse par dépression de l'air contenu dans le premier circuit et passant au travers de la buse.

L'invention a également pour objet un procédé d'un produit de désinfection à l'aide d'un dispositif de diffusion, caractérisé en ce qu'il comporte les étapes suivantes
- mettre en marche la pompe à air et une pompe d'amenée de produit de désinfection greffée sur le deuxième circuit, puis
- remplir complètement le premier circuit et le deuxième circuit respectivement en air et en produit,
- ouvrir la première électrovanne et la deuxième électrovanne, puis
- fermer la quatrième électrovanne, et
- arrêter le fonctionnement de la pompe à air et de la pompe d'amenée de produit.

L'invention sera mieux comprise à la lecture de la description qui suit et à l'examen des figures qui l'accompagnent. Celles-ci ne sont présentées qu'à titre indicatif et nullement limitatif de l'invention. Les figures montrent :

### Brève description des dessins

Figure 1 : Une représentation schématique d'un dispositif de diffusion, selon l'invention ;
Figure 2 : une représentation schématique d'une buse d'injection, selon l'invention, et
Figure 3 : une représentation graphique d'un procédé de désinfection de l'air à l'aide d'un dispositif de diffusion, selon l'invention.

### Description détaillée d'un mode de réalisation préférée de l'invention

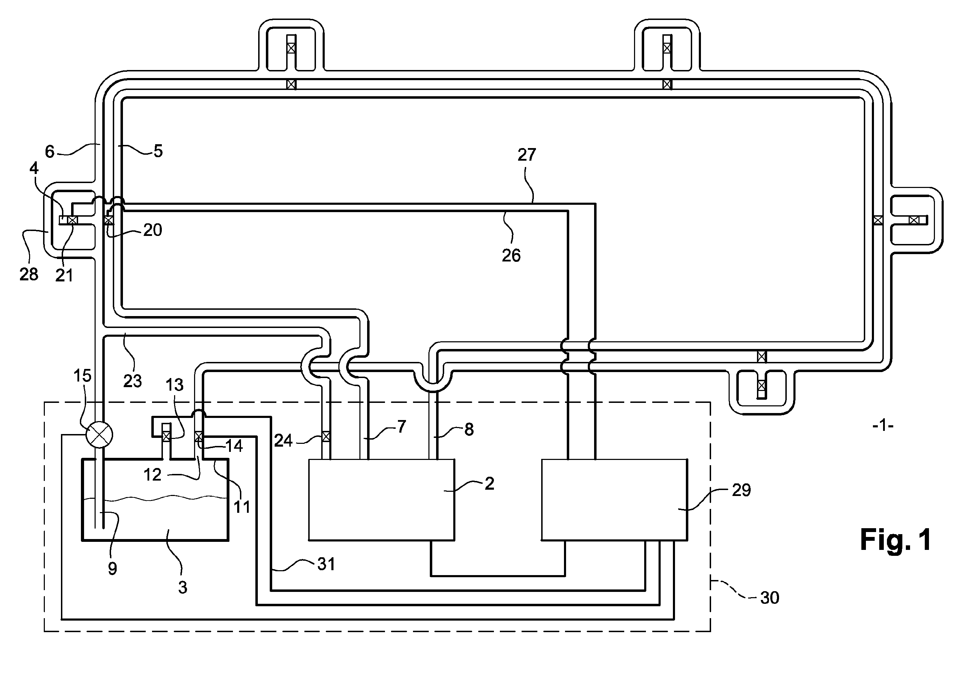

La figure 1 illustre un dispositif 1 de diffusion d'un produit de désinfection, selon l'invention. De préférence, la diffusion est une micro-diffusion. Par micro-diffusion, on entend une diffusion de particules sous la forme de particules microscopiques (10 à 30 µm). Dans une variante de l'invention, la diffusion s'effectue par diffusion de particules sous la forme de particules macroscopiques.

Le produit permet de désinfecter l'air ambiant ainsi que des surfaces potentiellement contaminées par des micro-organismes. Dans un exemple préféré, le produit présente des propriétés bactéricides, fongicides, non nocif pour les personnes susceptibles de l'inhaler après diffusion au moyen du dispositif de diffusion selon l'invention. Dans l'exemple préféré de l'invention, ce produit est sous forme liquide. En variante, ce produit pourrait être sous la forme de poudre.

Ce dispositif 1 comporte une pompe à air 2 pour produire de l'air comprimé, un réservoir de produit de désinfection 3, et au moins une buse d'injection 4. Dans l'exemple figure 1, le dispositif 1 comporte quatre buses telles que 4, distantes les unes par rapport aux autres.

L'air et le liquide sont mis au contact l'un de l'autre de manière à ce que l'air comprimé forme un moyen de propulsion du liquide au travers de la buse 4 afin de pulvériser le liquide sous forme de particules en suspension en sortie de la buse 4.

Selon l'invention, le dispositif 1 comporte également un premier circuit 5 et un deuxième circuit 6. Le premier circuit 5 forme un conduit relié à la pompe 2 à au moins une des buses 4. Le premier circuit est destiné à être rempli d'air. Le deuxième circuit 6 forme un autre conduit relié en boucle au réservoir de liquide 3 et est destiné à être rempli de liquide. Le premier circuit 5 et le deuxième circuit 6 sont raccordés l'un à l'autre par chacune des buses d'injection telles que 4. Pour simplifier la description, on décrira la buse 4 pour signifier chacune des quatre buses telles que 4.

La buse 4 reçoit donc l'air comprimé et le liquide de telle sorte que le liquide contenu dans le deuxième circuit 6 est expulsé vers l'extérieur au travers de la buse 4 par effet Venturi. La buse 4 comporte une première électrovanne 20 et une deuxième électrovanne 21. La première électrovanne 20 contrôle l'évacuation du liquide. La deuxième électrovanne 21 contrôle l'évacuation de l'air.

Le premier circuit 5 prend naissance à une première extrémité 7 par connexion avec la pompe à air 2 et finit dans un orifice d'une buse. Le premier circuit peut aussi se finir à une deuxième extrémité 8 opposée par connexion à la pompe 2. L'air circule à l'intérieur de ce premier circuit 5 dans un sens, de la première extrémité 7 vers la deuxième extrémité 8. Dans un exemple préféré de l'invention, la pression régnant à l'intérieur de ce premier circuit est de 4,5 KPa.

Le deuxième circuit 6 prend naissance à une première extrémité 9 en étant plongé dans le liquide contenu dans le réservoir 3 et finit à une deuxième extrémité 10 opposée de ce deuxième circuit 6 par connexion à une paroi 11 du réservoir de manière à ce qu'un orifice de passage 12 du conduit formé par ce deuxième circuit 6 débouche dans l'intérieur du réservoir 3. La liaison de la première extrémité 9 à la paroi 11 du réservoir 3 et la liaison de la deuxième extrémité 10 de ce deuxième circuit 6 à la paroi 11 du réservoir 3 sont étanches. Le liquide circule à l'intérieur du conduit formé par ce deuxième circuit 6 depuis la première extrémité 9 vers la deuxième extrémité 10 en se déversant dans le réservoir 3 par sa deuxième extrémité 10.

Le réservoir 3 est équipé d'une soupape 13 afin de libérer un éventuel excès d'air contenu dans le réservoir 3 et qui proviendrait du deuxième circuit 6 lors de son remplissage par le liquide.

Une troisième électrovanne 14 est disposée sur la deuxième extrémité 10 de ce deuxième circuit 6. Cette troisième électrovanne 14 permet de commander l'évacuation du liquide du deuxième circuit 6 le moment venu. Cette troisième électrovanne 14 permet de freiner et de régler le débit de liquide dans le conduit du deuxième circuit 6 pour garder une pression constante dans le deuxième circuit 6. En général, le temps de remplissage de ce deuxième circuit 6 est calculé pour que du liquide s'échappe au moins par cette deuxième extrémité 10 à l'intérieur du réservoir 3 avant que la troisième électrovanne 14 se ferme.

Pour remplir le deuxième circuit 6 en liquide, une pompe d'amenée 15 de produit est prévue à proximité du réservoir 3. La pompe 15 est branchée sur la première extrémité 9 de ce deuxième circuit 6 et permet de pomper le liquide provenant du réservoir 3 pour l'injecter dans le conduit formé par le deuxième circuit 6. Dans un exemple, le liquide présent dans le deuxième circuit 6 une fois rempli en ce liquide est à une pression de 1 KPa.

Selon l'invention, la pression régnant dans le premier circuit 5 est supérieure à la pression régnant dans le deuxième circuit 6 sauf aux endroits où est située la buse 4. A l'intérieur de la buse 4, la pression de l'air est réduite et est inférieure à la pression du liquide situé dans le deuxième circuit 6. Cette différence de pression permet de favoriser un appel de liquide vers l'extérieur via l'air contenu dans la buse 4. L'air contenu dans le buse 4 forme un moyen de propulsion du liquide vers l'extérieur par effet venturi.

Le conduit formé par le premier circuit 5 et le conduit formé par le deuxième circuit 6 sont placés parallèlement, l'un à côté de l'autre d'un point d'injection à un autre point d'injection. Chaque point d'injection est formé par une buse 4.

Le diamètre intérieur du conduit formé par le premier circuit 5 et le diamètre intérieur du conduit formé par le deuxième circuit 6 sont choisis de manière à ce que la durée de remplissage de chacun de ces conduits ne soit pas trop longue. En ce qui concerne plus particulièrement le deuxième circuit 6, on s'arrange pour qu'une faible quantité seulement de produit soit nécessaire pour remplir entièrement le conduit formé par le deuxième circuit 6 au cours de son remplissage. Ainsi, le diamètre intérieur de l'orifice du conduit du premier circuit 5 et le diamètre intérieur de l'orifice 12 du conduit du deuxième circuit 6 font chacun de 1 à 5 mm. Le diamètre intérieur du conduit du premier circuit 5 et le diamètre intérieur du conduit du deuxième circuit 6 sont identiques. En variante, le diamètre intérieur du conduit du premier circuit 5 et le diamètre intérieur du conduit du deuxième circuit 6 sont différents. Dans un exemple préféré de l'invention, le diamètre intérieur du conduit du premier circuit 5 et le diamètre intérieur du conduit du deuxième circuit 6 est de 2 mm.

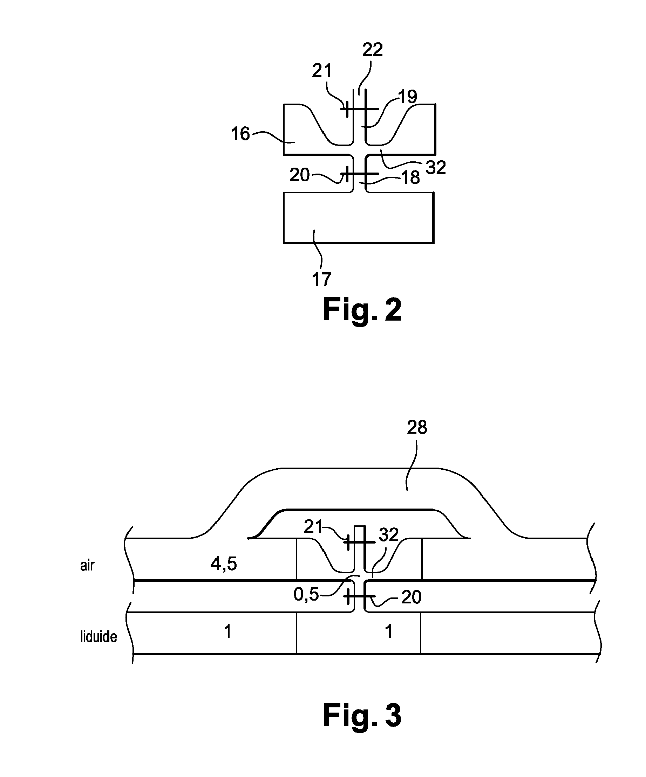

Une buse 4 est représentée schématiquement figures 2et 3. Cette buse 4 comporte un premier tube de raccordement 16 destiné à être branché en série sur le premier circuit 6. La buse 4 comporte un deuxième tube de raccordement 17 destiné à être branché en série sur le deuxième circuit 6. Le premier tube 16 communique avec le deuxième tube 17 par un canal de liaison 18. Le premier tube 16 forme un rétrécissement local central 32. Ce rétrécissement local 32 est de section plus petite que le reste de ce premier tube de raccordement 16. Le rétrécissement local 32 débouche à l'extérieur au moyen d'un conduit de sortie 19 de la buse 4 raccordé sur ce premier tube 16. Ainsi, l'air présent dans le premier tube 16 et situé à proximité du premier circuit 5 présente une pression plus élevée que celle de l'air présent dans le rétrécissement local 32 du premier tube 16 et éloigné du premier circuit 5. Dans un exemple, l'air situé proche du premier circuit 5 est à une pression identique à celle de l'air présent dans le premier circuit 5, c'est à dire 4,5KPa tandis que l'air présent dans le rétrécissement local 32 est à 0,5KPa. C'est la forme de ce premier tube 16 qui entraîne une diminution de pression ou un effet Venturi de la pression de l'air. La forme du premier tube 16 entraîne une chute de pression. Cette chute de pression est nécessaire à la propulsion du liquide depuis le deuxième circuit 6 vers l'extérieur via le canal 18, le premier tube 17 et le conduit de sortie 19 de la buse 4.

Le deuxième tube de raccordement 17 présente une section identique à celle du conduit formé par le deuxième circuit 6. Le premier tube 16, le deuxième tube 17 et le canal 18 débouchent tous dans le conduit de sortie 19 de la buse 4. La buse 4 permet le mélange de l'air et du liquide suivant le principe de Venturi.

La première électrovanne 20 est disposée sur le canal 18 reliant le premier tube 16 au deuxième tube 17. Cette première électrovanne 20 commande la sortie du liquide provenant du deuxième circuit 6. La deuxième électrovanne 21 est disposée sur le conduit de sortie 19, entre l'orifice de sortie 22 de la buse 4 et la première électrovanne 20. Cette deuxième électrovanne 21 commande la sortie de l'air provenant du premier circuit 5.

Un conduit de dérivation 28 ou conduit by-pass est branché en parallèle sur le premier circuit 5 aux endroits où est disposée une buse 4. Ce conduit de dérivation 28 contourne la buse 4 et permet de relier une première portion du premier circuit 5 à une deuxième portion de ce même premier circuit 5 afin d'assurer une continuité de passage de l'air au travers du premier circuit 5 malgré la présence de la buse 4. Ce conduit de dérivation 28 permet à l'air de contourner la buse 4 et d'être acheminé également tout le long du premier circuit 5 pour pouvoir également transiter dans autres buses installées tout le long du premier circuit 5 et du deuxième circuit 6.

Un tuyau d'amenée d'air 23 est relié à la pompe à air 2 et est destiné à être connecté au deuxième circuit 6, figure 1. Ce tuyau 23 est branché sur le deuxième circuit 6 en aval de la pompe 15. Une quatrième électrovanne 24 est disposée dans ce tuyau 23 pour commander une arrivée d'air depuis la pompe 2 vers le deuxième circuit 6.

Chaque électrovanne 20, 21, 14 et 24 est connectée à un fil électrique qui relie l'électrovanne correspondante à un boîtier de commande 29 gérant à distance l'ouverture de chacune de ces électrovannes. Un autre fil électrique relie le boîtier de commande 29 à la soupape 13. Dans l'exemple figure 1, pour simplifier la description et le dessin, seuls sont représentés les fils électriques 26, 27 et 31 connectés respectivement à la première électrovanne 20, à la deuxième électrovanne 21 et à la soupape 13. Le réservoir 3, la pompe à air 2, la pompe 15 et le boîtier de commande 29 sont placés dans un coffret unique 30. Ce coffret 30 peut être placé dans un local technique facile d'accès pour la maintenance.

L'orifice d'expulsion 22 de la buse 4 permet d'obtenir en sortie des particules de produit de diamètre mesurant entre 10 et 30 µm. Dans un exemple préféré de l'invention, l'orifice de la buse présente un orifice d'expulsion permettant d'obtenir une particule de produit de diamètre 15 µm. Un tel diamètre assure une diffusion homogène du liquide dans l'air.

Le dispositif 1 peut être disposé à l'extérieur d'une gaine de ventilation afin que les buses 4 injectent le liquide au travers de la gaine et que le liquide se retrouve à l'intérieur de la gaine. Le flux d'air présent dans la gaine transporte alors le liquide diffusé par les buses tout le long de la gaine. Dans un exemple, lorsque l'on vaporise le liquide à 15 µm, à 10m/s, on retrouve des traces de ce liquide en suspension dans la gaine jusqu'à 200m du point d'injection. Une telle disposition du dispositif à l'extérieur de la gaine permet de désinfecter les conduits d'amenées d'air en général d'un bâtiment ou d'un immeuble.

L'air est recyclé dans les conduits d'aération donc au bout d'un moment on a aussi du produit dans les conduits d'aération quand le cycle est établi, le produit en suspension.

Ou bien, le dispositif peu être placé au dessus d'un plafond afin que les buses injectent le liquide au travers du plafond dans la pièce située en dessous. Dans une pièce, on peut retrouver de ce liquide jusqu'à 3 m du point de pulvérisation.

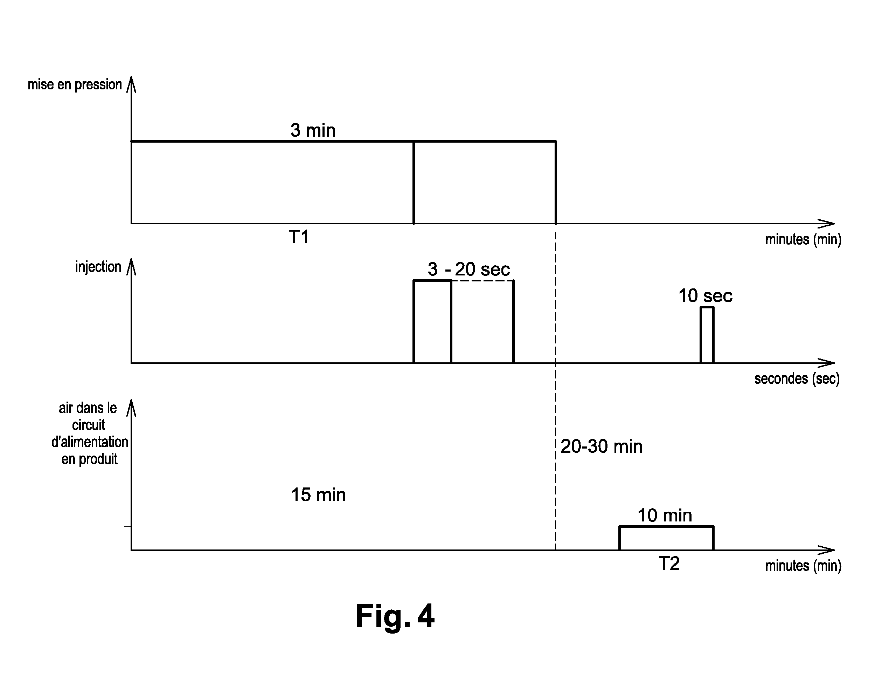

Figure 4, le procédé de diffusion du liquide est réalisé de manière automatique et de la manière suivante. Une première phase T1 de mise en pression de l'air et du liquide consiste à mettre en marche la pompe à air 2 et la pompe 15 pour remplir le premier circuit 5 et le deuxième circuit 6 respectivement en air comprimé à débit constant et en liquide à débit constant. Le liquide est très peu mis en pression, 1 KPa, c'est juste pour charger le circuit.

Pendant 3 minutes, le premier circuit 5 est rempli d'air jusqu'à ce que l'air atteigne une pression par exemple de 4,5 KPa et le deuxième circuit 6 est rempli de liquide jusqu'à ce que le liquide atteigne une pression par exemple de 1 KPa. Puis pendant 3 à 20 secondes et pour au moins l'une des buses 4, la première électrovanne 20 et la deuxième électrovanne 21 souvrent simultanément afin de pulvériser le produit dans l'air. Quand on ouvre les électrovannes 20 et 21, c'est l'air comprimé qui sert de moteur de diffusion du liquide. Puis on déclenche la fermeture de la première électrovanne 20 et la deuxième électrovanne 21. Puis, au bout de quelque secondes, la pompe 15 et la pompe 2 s'arrêtent.

Si un autre ordre de diffusion du produit n'est pas commandé au bout d'un certain temps après la phase T1, une deuxième phase T2 de nettoyage est mise en place. La phase T2 est mise en place au minimum avant que le liquide ait eu le temps de sécher dans le conduit du deuxième circuit 6 et dans les buses 4 et avant que le liquide n'obstrue le conduit du deuxième circuit 6 ainsi que l'orifice de sortie 22 de la buse 4.

En général, une durée de 10 à 30 minutes maximum est fixée après la fin de la phase T1 pour débuter la phase T2. Dans un exemple préféré de l'invention, au bout de 20 minutes, la phase T2 est mise en place si un nouveau cycle de phase T1 n'est pas mis en place. Pendant la deuxième phase T2, la pompe à air 2 est mise en marche et la quatrième électrovanne 24 s'ouvre permettant à l'air comprimé de vidanger le conduit du deuxième circuit 6 puis de circuler au travers du tuyau 23 puis au travers du deuxième circuit 6. Cette phase T2 peut durer entre 5 à 15 minutes. Avant la fin de cette phase T2, on enclenche l'ouverture et la fermeture de la première électrovanne 20, de la deuxième électrovanne 21 et de la troisième électrovanne 14 afin de faire transiter l'air sous pression au travers de ces mêmes électrovannes 20, 21 et 14 pour les nettoyer.

Il est prévu de procéder à ce nettoyage au moins une fois par jour minimum. Dans une variante, ce nettoyage est automatiquement réalisé si la première électrovanne 20, la deuxième électrovanne 21 et la troisième électrovanne 14 ne sont pas ouvertes dans la demi-heure suivant la dernière fermeture de ces mêmes électrovannes 20 et 21.

Le dispositif de micro-diffusion est autonome. Le produit utilisé est pur, non dilué. On fait véhiculer le moins de produit possible pour ne pas mettre trop grosse quantité de produit de désinfection. En variante, le produit peut être dilué. La quantité de produit de désinfection dépend de sa concentration et des besoins hygiéniques de l'établissement à traiter (maison de retraite, hôpital, chambres d'hôtel, immeubles de bureaux....)

Dans un exemple, une armoire métallique ou coffret 30 peut contenir un réservoir 3 de 50 litres de produit de désinfection par an pour la consommation de tout un bâtiment de 4 000 m2, en traitant 8 fois 5 secondes par jour, avec 3 jeux de deux circuits tels que 5 et 6 différents, chacun des jeux comportant quatre buses telles que 4.

On régule la pression de l'air tout en l'asséchant, en la déshumidifiant, on élimine les éventuelles huiles provenant du compresseur de la pompe.

Dans une variante, on dispose un détecteur de niveau relié électriquement au boîtier de commande 29.

Dans un exemple, le premier circuit 5 mesure 150 m de long, avec un diamètre intérieur du conduit de ce premier circuit 5 de 2 mm. Quelque soit la longueur du premier circuit 5, il faut prévoir 30 litres par minutes par buse d'air comprimé pour remplir entièrement le conduit formé par ce premier circuit 5.

Dans un autre exemple, le deuxième circuit 6 mesure 150 m de long, avec un diamètre intérieur du conduit de ce deuxième circuit 6 de 2 mm, il faut prévoir 3 litres de liquide pour remplir entièrement le conduit formé par ce deuxième circuit 6.

## Revendications

1. Dispositif de diffusion (1) d'un produit de désinfection, comportant
- une pompe à air (2) pour produire de l'air comprimé,
- un réservoir (3) de produit de désinfection, et
- au moins une buse d'injection (4),
**caractérisé en ce que** le dispositif comporte également
- un premier circuit (5) et un deuxième circuit (6), le premier circuit formant un conduit relié à la pompe et débouchant dans la buse et destiné à être rempli d'air et le deuxième circuit formant un autre conduit relié en boucle au réservoir de produit et destiné à être rempli de produit, le premier circuit et le deuxième circuit étant raccordés l'un à l'autre par au moins une buse d'injection, la buse recevant l'air comprimé et le produit de telle sorte que le produit contenu dans le deuxième circuit est expulsé vers l'extérieur au travers de la buse par dépression de l'air contenu dans le premier circuit et passant au travers de la buse.

2. Dispositif selon la revendication 1, **caractérisé en ce qu'**il comporte une première électrovanne (20) et une deuxième électrovanne (21) branchées sur la buse, la première électrovanne commandant l'arrivée d'air par le premier circuit et la deuxième électrovanne commandant l'arrivée de produit par le deuxième circuit.

3. Dispositif selon l'une des revendications 1 à 2, **caractérisé en ce que** le premier circuit comporte une portion de conduit de dérivation (28) greffée sur le conduit formé par le premier circuit, ladite portion de conduit contournant la buse.

4. Dispositif selon l'une des revendications 1 à 3, **caractérisé en ce que** le conduit formé par le premier circuit a un diamètre intérieur de 1 à 5 mm.

5. Dispositif selon l'une des revendications 1 à 4, **caractérisé en ce que** le conduit formé par le deuxième circuit a un diamètre intérieur de 1 à 5 mm.

6. Dispositif selon l'une des revendications 1 à 5, **caractérisé en ce que** la buse présente un orifice d'expulsion (22) de produit permettant de produire une particule expulsée de diamètre de 10 à 30 µm.

7. Dispositif selon l'une des revendications 1 à 6, **caractérisé en ce qu'**il est placé à l'extérieur d'une gaine de ventilation afin d'injecter le liquide au travers de cette même gaine.

8. Dispositif selon l'une des revendications 1 à 7, **caractérisé en ce qu'**il est disposé au-dessus d'un plafond.

9. Procédé de diffusion d'un produit de désinfection à l'aide d'un dispositif de diffusion selon l'une des revendications précédentes, **caractérisé en ce qu'**il comporte les étapes suivantes
- mettre en marche la pompe à air et une pompe d'amenée (15) de produit de désinfection greffée sur le deuxième circuit, puis
- remplir complètement le premier circuit et le deuxième circuit respectivement en air et en produit,
- ouvrir la première électrovanne et la deuxième électrovanne, puis
- fermer la première électrovanne et la deuxième électrovanne, et
- arrêter le fonctionnement de la pompe à air et de la pompe d'amenée de produit.

10. Procédé selon la revendication 9, **caractérisé en ce qu'**il comporte les étapes suivantes
- ouvrir une quatrième électrovanne (24) située sur un conduit d'amenée d'air (23) reliée à la pompe à air débouchant dans le deuxième circuit, puis
- mettre en marche la pompe à air,
- remplir le deuxième circuit d'air comprimé, puis
- ouvrir simultanément la première électrovanne et la deuxième électrovanne, puis
- fermer la première électrovanne et la deuxième électrovanne, puis
- fermer la quatrième électrovanne, et
- arrêter le fonctionnement de la pompe à air et fermer la quatrième électrovanne.

11. Procédé selon la revendication 10, **caractérisé en ce qu'**il comporte l'étape suivante
- réaliser les étapes selon la revendication 10 une fois par jour.
